# EUROPEAN PATENT APPLICATION

(11) **EP 2 255 767 A1**
(43) Date of publication of application: **01.12.2010**
(21) Application number: 09724602.9
(22) Date of filing: 19.01.2009
(51) Int. Cl.: A61F 13/49, A61F 13/15, A61F 13/494, A61F 13/511

(54) **DISPOSABLE PANTS-TYPE DIAPER**

(30) Priority: 26.03.2008 JP 2008081758
(71) Applicant: Uni-charm Corporation, Ehime 799-0111 (JP)
(72) Inventor: OTSUBO, Toshifumi, Kanonji-shi Kagawa 769-1602 (JP)
(74) Representative: Knights, Rupert
(86) International application number: PCT/JP2009/050616
(87) International publication number: WO 2009/119137

(57) **Abstract**

The present invention aims to improve a leak-barrier effect of the disposable pants-type diaper provided with a separator serving to prevent urine and feces from being mixed with each other.

A disposable pants-type diaper has the innermost sheet member (21) interposed between a liquid-pervious inner sheet (23) covering a bodily fluid-absorbent structure (22) and the diaper's skin. The innermost sheet member (21) is formed with a front opening (31) and a rear opening (32) communicating with a body waste retaining space (25) defined between the inner sheet (23) and the innermost sheet member (21). A portion of the innermost sheet member (21) extending between these two openings (31, 32) is folded and joined together in a pair of joints arranged on a vertical center line (P-P) of the diaper and extends downward toward the inner sheet (23) to form a separator. The innermost sheet member (21) is provided between a top (31t) of the front opening (31) and the associated one of the joints on the center line (P-P) and between a top (32t) of the rear opening (32) and associated one of the joints on the center line (P-P) with elastic regions (71, 72), respectively, adapted to be contractible in a transverse direction (B), on one hand, and with a non-elastic region (73) between the elastic regions (71, 72), on the other hand.

## Description

### TECHNICAL FIELD

The present invention relates to disposable pants-type diapers and more particularly to such diapers each provided with a separator functioning to prevent urine and feces from being mixed with each other.

### RELATED ART

In disposable pants-type diapers, it is known to provide its crotch region with means functioning to prevent urine and feces from being mixed with each other. For example, the pants-type diaper disclosed in JP 2002-11044 A (PATENT DOCUMENT 1) includes a skin-contact sheet provided on a skin-facing side of a liquid-pervious inner sheet covering an absorbent structure. In its crotch region, a portion of the skin-contact sheet adapted to be spaced upward from the inner sheet is formed with an opening for passage of urine or feces. Elastics are attached under tension to the skin-contact sheet so as to surround this opening. In addition, the crotch region is provided with dam-like means serving to prevent urine and feces from being mixed with each other. An absorbent pant disclosed in JP 10-513071 T (PATENT DOCUMENT 2) comprises a pant-layer, an outer barrier-layer provided on the outer side of the pant-layer and an absorbent structure sandwiched between the pant-layer and the outer barrier-layer. In the crotch region, the pant-layer is formed with an opening for passage of urine or feces toward the absorbent structure. The pant-layer is made in a manner that the crotch region is formed in a stretched state under tension in the longitudinal direction while the front and rear waist regions thereof are formed in a stretched state under tension in the transverse direction. In the pant-layer, the elasticity of the front and rear waist regions primarily functions in the circumferential direction of the waist-opening.
PATENT DOCUMENT 1: JP 2002-11044 A
PATENT DOCUMENT 2: JP 10-513071 T

### DISCLOSURE OF THE INVENTION

### PROBLEM TO BE SOLVED BY THE INVENTION

The skin-contact sheet of the diaper disclosed in PATENT DOCUMENT 1 has its front and rear ends fixed to the inner sheet in the front and rear waist regions, respectively, so that the intermediate section defined between the front and rear ends is not fixed to the inner sheet and suspended. With the diaper having such arrangement put on the wearer's body, the skin-contact sheet may be unacceptably displaced or deformed in the crotch region. If the displacement of the skin-contact sheet exceeds the acceptable range, the opening of the skin-contact sheet may become out of alignment with the wearer's external genital or anus and the dam-like means to serve as a separator will be unable to fulfill its primary function. The pants disclosed in PATENT DOCUMENT 2 have not a separator serving to prevent urine and feces from being mixed with each other but the pant-layer formed with the opening is able to prevent from a mixture of urine and feces from contacting with the wearer's skin as long as the pant-layer is kept in close contact with the wearer's skin. However, the pant-layer is elastic and may cause some problems in the crotch region depending on the direction in which the pant-layer is contracted in the crotch region. Specifically, if the crotch region of the pant-layer is formed in a stretched state under tension in the longitudinal direction, it will be difficult for the crotch region to be contracted circumferentially around the respective leg-openings and to come in close contact with the wearer's thighs. In order that the crotch-region is contractible circumferentially of the respective leg-openings, the crotch region of the pant-layer must be formed in a stretched state under tension in the transverse direction and such crotch region necessarily behaves to contract in the transverse direction. With the pant having such crotch region put on the wearer's body, the crotch region may come off from the wearer's inguinal region. From the viewpoint of the preventive effect against sideways leakage of bodily fluids from the pant, such potential movement of the crotch region is generally undesirable.

In view of the problem as has been described above, it is a principal object of the present invention to improve the disposable pants-type diaper provided with a separator serving to prevent urine and feces from being mixed with each other.

### MEASURE TO SOLVE THE PROBLEM

The object set forth above is achieved, according to the present invention, by an improvement in a disposable pants-type diaper having a front-back direction, a transverse direction and a vertical direction extending orthogonally one to another, a crotch region, a front waist region extending forward from the crotch region and a rear waist region extending rearward from the crotch region wherein the crotch region includes a bodily fluid-absorbent structure comprising a bodily fluid-absorbent core sandwiched between a liquid-pervious inner sheet and a liquid-impervious outer sheet and the disposable pants-type diaper is formed in the crotch region with a separator serving to separate urine and feces from each other.

The improvement according to the present invention is **characterized in that** the bodily fluid-absorbent structure has side flaps formed outboard of the core in the transverse direction and end flaps formed outboard of the core in the vertical direction. The innermost sheet member joined to the side flaps and the end flaps so as to be interposed between the pants-type diaper's skin and the inner sheet and to extend in the crotch region and the front and rear waist regions is partially spaced from the inner sheet in the vertical direction to define a body waste retaining space and the innermost sheet member is formed with a front opening allowing urine to flow into the body waste retaining space and a rear opening allowing feces to move into the body waste retaining space. A region of the innermost sheet member extending between the front opening and the rear opening is folded along a fold line extending in the transverse direction and defined by a center line bisecting a dimension of the diaper in the transverse direction with a skin-facing surface of the innermost sheet member inside so that the region extending between the front and rear opening and folded to face itself and partially bonded together and the innermost sheet member extends downward from this bonded region toward the inner sheet in the vertical direction to form the separator. The innermost sheet member further includes elastic regions adapted to be elastically contractible and respectively defined between the region facing itself in the front-back direction and partially bonded together and a top of the front opening as viewed in the vertical direction and between the region facing itself in the front-back direction and partially bonded together and a top of the rear opening as viewed in the vertical direction, on one hand, and a non-elastic region defined by a portion extending downward from the bonded regions, on the other hand.

According to one preferred embodiment of the present invention, the elastic regions extend in the transverse direction in the crotch region and joined under tension to the side flaps of the bodily fluid-absorbent structure.

According to another preferred embodiment of the present invention, the innermost sheet member is pant-shaped.

According to still another preferred embodiment of the present invention, the side flaps are provided with elastic members extending in the front-back direction attached under tension thereto and the elastic regions intersect and overlap the elastic members.

According to yet another preferred embodiment of the present invention, the elastic regions and the elastic members are bonded together in regions of intersecting and overlapping.

### EFFECT OF THE INVENTION

In the disposable pants-type diaper according to the present invention, the innermost sheet member is formed with the front opening and the rear opening and, in the crotch region, the portion of the innermost sheet member extending between these two openings is folded downward and partially joined together to form the separator. The separator serves to prevent urine passing through the front opening and feces passing through the rear opening from being mixed with each other on the liquid-pervious inner sheet. The innermost sheet member further includes elastic regions adapted to be elastically contractible and respectively defined between the region facing itself in the front-back direction and partially bonded together and a top of the front opening as viewed in the vertical direction and between the region facing itself in the front-back direction and partially bonded together and a top of the rear opening as viewed in the vertical direction, on one hand, and a non-elastic region defined by a portion extending downward from the bonded regions, on the other hand. With such unique arrangement, with the diaper put on the wearer's body, the presence of the innermost sheet member would not contract the crotch region in the transverse direction to disengage the side edges of the crotch region from the wearer's inguinal region.

The embodiment of the present invention wherein the innermost sheet member is pant-shaped facilitates the front and rear openings to be aligned with the wearer's external genital and anus, respectively, when the diaper is put on the wearer's body.

In the embodiment of the present invention wherein the elastic regions adapted to be contractible in the transverse direction intersect with the elastic members provided along the side flaps of the bodily fluid-absorbent structure, these elastic regions cooperate with the elastic members to assure reliable fitness of the diaper around the wearer's thighs.

In the embodiment of the present invention wherein the innermost sheet member and the side flaps are bonded together in the regions of intersecting and overlapping, the side flaps are smoothly curved along the side edges of the core so that the side flaps may be smoothly put in contact with the wearer's inguinal region.

### BRIEF DESCRIPTION OF THE DRAWINGS

[FIG. 1] Fig. 1 is a partially cutaway perspective view of a disposable pants-type diaper according to the present invention.
[FIG. 2] Fig. 2 is a sectional view taken along the line II-II in Fig. 1.
[FIG. 3] Fig. 3 is a partially cutaway plan view of the diaper having been flatly developed.
[FIG. 4] Fig. 4 is a view similar to Fig. 3, illustrating one preferred embodiment of the present invention.

### IDENTIFICATION OF REFERENCE NUMERALS USED IN THE DRAWINGS

- 1: pants-type diaper
- 6: crotch region
- 7: front waist region
- 8: rear waist region
- 21: innermost sheet member
- 22: bodily fluid-absorbent structure
- 22a: side flaps
- 23a: end flaps
- 23: inner sheet
- 24: outer sheet
- 25: body waste retaining space
- 25a: body waste retaining space
- 25b: body waste retaining space
- 31: opening
- 31t: opening's top
- 32: opening
- 32t: opening's top
- 36: separator
- 37: joint (joint region)
- 71: elastic region
- 72: elastic region
- 73: non-elastic region
- A: front-back direction
- B: transverse direction
- C: vertical direction
- Q-Q: folding line (transverse center line)
- P-P: center line (longitudinal center line)

### DETAILED DESCRIPTION OF THE PREFERRED EMBODIMENTS

Details of a disposable pants-type diaper according to the present invention will be more fully understood from the description given hereunder in reference to the accompanying drawings.

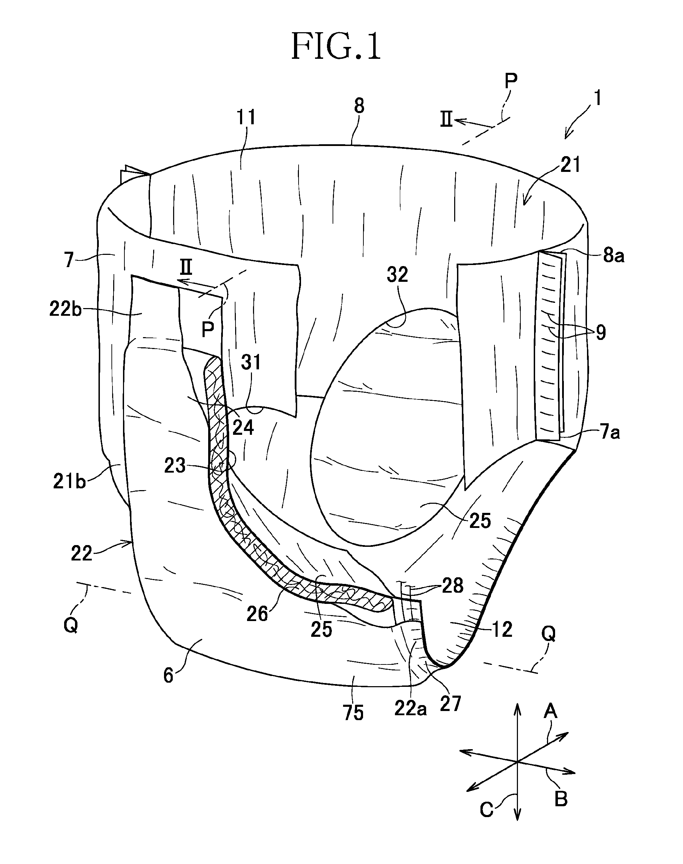

Fig. 1 is a partially cutaway perspective view of the disposable pants-type diaper 1 put on the wearer's body wherein a front-back direction, a transverse direction and a vertical direction are indicated by double-headed arrows A, B and C extending orthogonally one to another. The diaper 1 has a crotch region 6, a front waist region 7 extending forward from the crotch region 6 and a rear waist region 8 extending rearward from the crotch region 6 wherein respective side edges of the front and rear waist regions 7, 8 are put flat and sealed together at joints 9 arranged intermittently in the vertical direction C to form a waist-opening 11 and a pair of leg-openings 12. In addition, the diaper 1 includes the innermost sheet member 21 facing the diaper's skin (not shown) and a bodily fluid-absorbent structure 22 attached to the outer surface 21b of the innermost sheet member 21. Of the innermost sheet member 21, the respective portions defining the front and rear waist regions 7, 8 are elastically contractible in the transverse direction B and most of the portion defining the crotch region 6 is not contractible in the transverse direction B elastically as well as non-elastically. While the innermost sheet member 21 is preferably liquid-impervious over all and more preferably air-permeable and liquid-impervious over all, it is possible to form the innermost sheet member 21 in such a manner that the portions defining the front and rear waist regions 7, 8, respectively, are liquid-impervious and the portion defining the crotch region 6 is liquid-pervious.

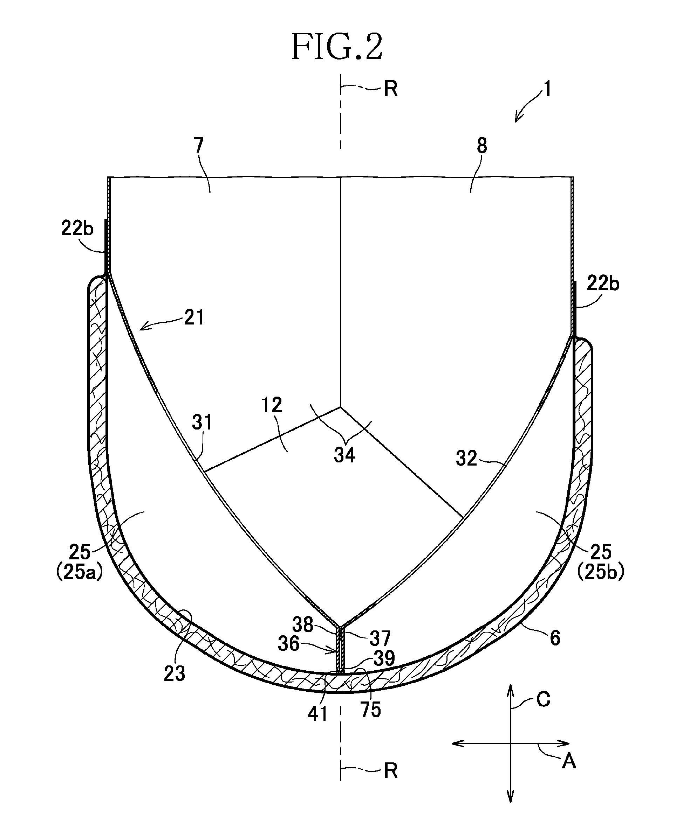

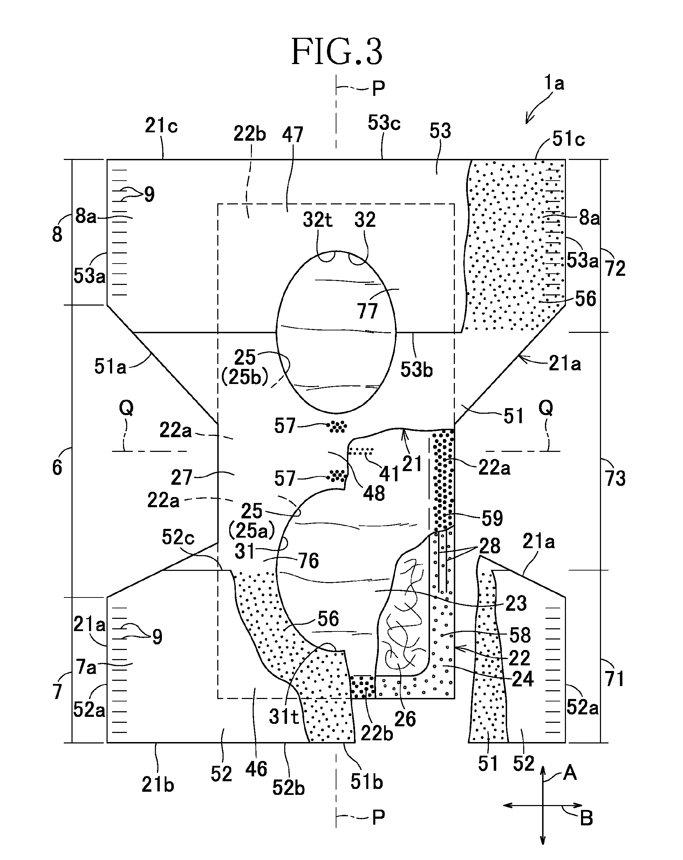

Referring to Fig. 1, the bodily fluid-absorbent structure 22 comprises a liquid-pervious inner sheet 23, a liquid-impervious outer sheet 24 and a core 26 sandwiched between these two sheets 23, 24 wherein the core 26 comprises, in turn, a bodily fluid-absorbent material such as fluff pulp fibers wrapped with a tissue paper. These two sheets 23, 24 extend outward from a periphery of the core 26 are bonded to each other outboard of the periphery to form a pair of side flaps 22a and a pair of end flaps 22b. These side flaps 22a and end flaps 22b are bonded to the innermost sheet member 21 (See Figs. 2 and 3). In the crotch region 6, for example, the side flaps 22b and the innermost sheet member 21 are put flat together to define side edges 27. Along these side edges 27, leg elastic members 28 are sandwiched between the inner and outer sheets 23, 24 and bonded under tension to at least one of these sheets 23, 24 to define respective parts of leg elastic regions. In a region extending in the transverse direction B between the side edges 27, 27, the inner sheet 23 and the innermost sheet member 21 are spaced from each other in the vertical direction C to form a body waste retaining space 25. The end flaps 22b of the bodily fluid-absorbent structure 22 are bonded to the outer surface 21b of the innermost sheet member 21 and therefore no more function as the flaps.

The innermost sheet member 21 used in such manner is formed before the transverse center line Q-Q extending in the transverse direction B to bisect a dimension of the diaper 1 in the front-back direction A with a front opening 31 and behind the transverse center line B with a rear opening 32. The front opening 31 is positioned so that the wearer's external genital can be exposed therein and the rear opening 32 is positioned so that the wearer's anus can be exposed therein. In this way, urine and feces excreted by the wearer directly flow or move into the body waste retaining space 25 not through the innermost sheet member 21. Urine and feces having flown or moved in into the body waste retaining space 25 is retained within the space defined between the innermost sheet member 21 and the inner sheet 23 spaced from each other and thereby prevented from coming in contact with the wearer's skin. In addition, the inner sheet 23 having absorbed such urine or feces and being in wet condition is covered with the innermost sheet member 21. The innermost sheet member 21 thus protects the wearer from uncomfortable feeling which would otherwise be experienced by the wearer due to contact with the wet inner sheet 23.

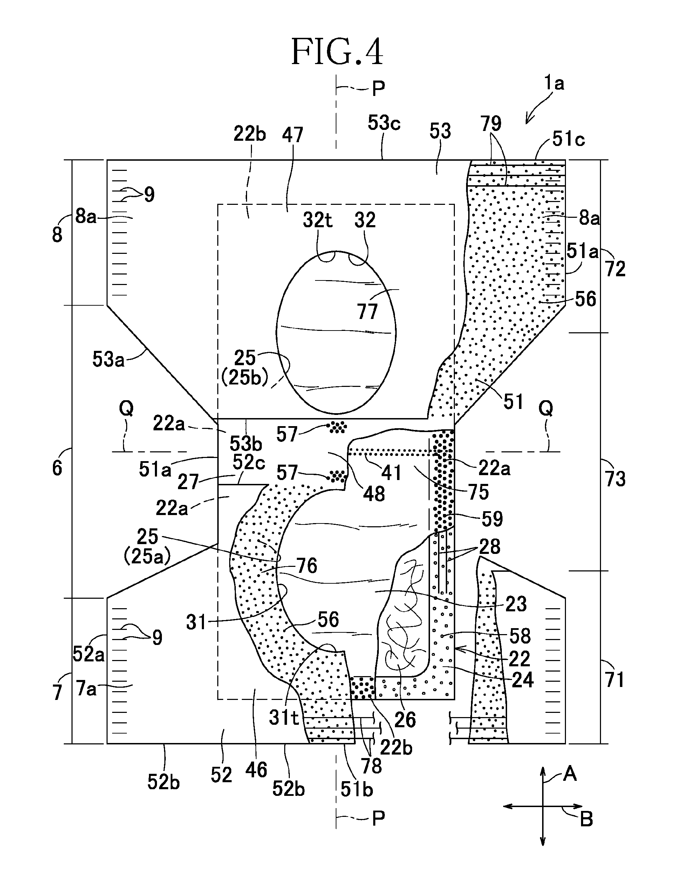

Fig. 2 is a sectional view taken along the line II-II in Fig. 1 wherein the line II-II corresponds to the central line P-P extending in the front-back direction A to bisect a dimension of the diaper 1 in the transverse direction B. The portions of the innermost sheet member 21 in the front and rear waist regions 7, 8, respectively, are bonded to each other in parallel to the vertical center line R-R extending in the vertical direction C and bisecting the dimension of the diaper 1 in the front-back direction A to define upper segments of peripheral edges surrounding the respective leg-openings 12. In the crotch region 6, the innermost sheet member 21 is formed with a tuck 36. The tuck 36 is formed by bonding or sealing regions of the innermost sheet member 21 facing each other along the vertical center line R-R at a joint 37. More specifically, the innermost sheet member 21 is folded along the vertical center line R-R in its region between the front opening 31 and the rear opening 32 with the skin-facing inner surface of the innermost sheet member 21 inside. In other words, the tuck 36 extends downward from the portion of the innermost sheet member 21 spaced from the inner sheet 23 toward the inner sheet 23 to lie on the transverse center line Q-Q (See Fig. 1). While the joint 37 for the tuck 36 is illustrated to be formed at least along the uppermost segment 38 of the tuck 36 as viewed in the vertical direction C, the joint 36 may be enlarged by an appropriate dimension toward a lower end 39 of the tuck 36. While a dimension of the joint 37 in the transverse direction B is principally smaller than a width dimension of the crotch region 6, the dimension of the joint 37 may be enlarged unless the joint 37 prevents the diaper 1 from being smoothly put on the wearer's body. For example, in the case of the diaper 1 exclusively for baby, the dimension of the joint 37 in the transverse direction B is preferably in a range of 10 to 70mm. The lower end 39 of the tuck 36 may be coated on its outer surface with joining means such as adhesive and joined to the inner sheet 23. In this case, the lower end 39 may be joined only along a middle portion thereof in the transverse direction B or over all to the inner sheet 23 (See Fig. 4).

Between the innermost sheet member 21 and the inner sheet 23, the body waste retaining space 25 divided into a front half and a rear half by the tuck 36. Specifically, the body waste retaining space 25 is divided into a front body waste retaining sub-space 25a defined before the transverse center line Q-Q and a rear body waste retaining sub-space 25b defined behind the transverse center line Q-Q. Between these two sub-spaces 25a, 25b, the tuck 36 extends upward from the inner sheet 23. The front opening 31 of the innermost sheet member 21 communicates with the inside and the outside of the front body waste retaining sub-space 25a and lets urine flow thereinto. The rear opening 32 communicates with the inside and the outside of the rear body waste retaining sub-space 25b and legs feces move thereinto.

Fig. 3 is a partially cutaway plan view showing the diaper 1a corresponding to the diaper 1 of Figs. 1 and 2 after the front and rear waist regions 7, 8 have been unjoined from each other at the joints 9, the respective halves of the innermost sheet member 21 have been unjoined from each other at the joint 37 for the tuck 36 and then the crotch region 6 and the front and rear waist regions 7, 8 have been developed in the front-back direction A as well as in the transverse direction B. It should be noted here that the regions in the flatly developed diaper 1a which are common to those in the diaper 1 are designated by same reference numerals as those used for the diaper 1.

The innermost sheet member 21 is contoured by a pair of side edges 21a, a front end 21b and a rear end 21c in a concave shape curved inwardly and comprises a first sheet 51 defining at least a central portion of the crotch region 6 as viewed in the front-back direction A, a second sheet 52 defining at least the front waist region 7 and a third sheet 53 defining at least the rear waist region 8 arranged in the front-back direction A. The first sheet 51 is not elastically contractible and faces the inner sheet 23 according to the embodiment of Fig. 3 and the same as the innermost sheet member 21 in shape and dimension. In other words, side edges, front end and rear end of the first sheet 51 correspond to the side edges 21a, the front end 21b and the rear end 21c of the innermost sheet member 21. The second sheet 52 is elastically contractible in the transverse direction B and bonded under tension in the transverse direction B to the inner surface of the first sheet 51 by hot melt adhesive 56 according to the embodiment of Fig. 3. The second sheet 52 is contoured by a pair of side edges 52a, a front end 52b and a rear end 52c wherein the side edges 52a and the front ends 52b substantially correspond to the side edges 51a and the front end 51b, respectively. The rear end 52c of the second sheet 52 lies in the crotch region 6 and extends across the front opening 31. The third sheet 53 also is elastically contractible in the transverse direction B and, according to the embodiment of Fig. 3, bonded under tension in the transverse direction B to the inner surface of the first sheet 51 by hot melt adhesive 56. The third sheet 53 is contoured by a pair of side edges 53a, a front end 53b and a rear end 53c wherein the side edges 53a and the rear end 53c substantially correspond to the side edges 511a and the rear end 51c of the first sheet 51. The front end 53b of the third sheet 53 lies in the crotch region 6 and extends across the rear opening 32. In this way, the innermost sheet member 21 is formed with front and rear elastic regions 71, 72 both adapted to be elastically contractible in the transverse direction B under the effect of the second sheet 52 and the third sheet 53, respectively. Between the rear end 52c of the second sheet 52 and the front end 53b of the third sheet 53 as viewed in the front-back direction A of the innermost sheet member 21, a non-elastic region 73 which is not elastically contractible in the transverse direction B is formed. A pair of regions 57 each comprising a plurality of dots gathered together on the inner surface of the first sheet 51 corresponds to the joints 37 in Fig. 2 located symmetrically about the transverse center line Q-Q.

In the bodily fluid-absorbent structure 22, the inner sheet 23 and the outer sheet 24 sandwiching therebetween the core 26 extend outward from the periphery of the core 26 and bonded to each other outboard of the periphery of the core 26 by hot melt adhesive 58 to form the side flaps 22a and the end flaps 22b. Along the crotch region's side edges 27 in the flatly developed diaper 1a, the leg elastic members 28 are sandwiched between the inner sheet 23 and the outer sheet 24 and extend under tension in the front-back direction A. Portions of the inner sheet 23 are bonded to the outer surface 21b of the innermost sheet member 21 by hot melt adhesive 59. The outer sheet 24 is preferably the same as the inner sheet 23 in shape as well as in size or dimensioned to slightly larger than the inner sheet 23. When the outer sheet 24 is dimensioned to be slightly larger than the inner sheet 23, a portion of the outer sheet 24 extending outward from the periphery of the inner sheet 23 is more preferably bonded to the outer surface 21b of the innermost sheet member 21.

To obtain the diaper 1 from the flatly developed diaper 1a of Fig. 3, the flatly developed diaper 1a is folded along the transverse center line Q-Q with the innermost sheet member 21 inside, then respective halves of the first sheet 51 are joined to each other by joining the two regions 57 to each other and simultaneously the side edges 7a, 7a of the front waist region 7 defined by the innermost sheet member 21 are joined to the side edges 8a, 8a of the rear waist region 8 defined also by the innermost sheet member 21 at the joints 9 (See Fig. 1).

With the diaper 1 obtained in this manner being put on the wearer's body, the innermost sheet member 21 in the crotch region 6 is spaced upward from the portion of the inner sheet 23 surrounded by the side flaps 22a and the end flaps 22b of the bodily fluid-absorbent structure 22 and thereby forms the body waste retaining space 25 adapted to prevent urine and feces from coming in contact with the wearer's skin. At the same time, the innermost sheet member 21 in the crotch region 6 functions as a barrier adapted to prevent the inner sheet 23 soiled and/or wetted with feces and/or urine from coming in contact with the wearer's skin. Furthermore, the portion of the innermost sheet member 21 defining the tuck 36 cooperates with the portions of the innermost sheet member 21 extending outward from both sides of the tuck 36 in the transverse direction B to form a partition serving as a separator adapted to prevent urine and feces from being mixed with each other. As a consequence, in this diaper 1, fluidity of body waste would not be enhanced due to intermixture of feces and urine and the wearer's skin would not be soiled with body waste over a wide range. Furthermore, a length dimension of the innermost sheet member 21 extending along the center line P-P extending in the front-back direction A as viewed in Fig. 3 is shortened by the dimension used to form the tuck 36 as seen in Figs. 1 and 2 and therefore the innermost sheet member 21 would not be formed with remarkable gathers. The second and third sheets 52, 53 of the innermost sheet member 21 formed with the front elastic region 71 and the rear elastic region 72, respectively, are present in the front and rear waist regions 7, 8, respectively, and thereby assure high fitness of these two waist regions 7, 8 to the wearer's skin. Portions of the second and third sheets 52, 53 forming parts of the crotch region 6 form at least parts of the respective peripheries 76, 77 of the front and rear openings 31, 32 of the first sheet 51 serve to assure elastic fitness of these peripheries to the wearer's skin around the wearer's external genital and anus and reliable flow or movement of urine or feces into the body waste retaining space 25.

As will be apparent from the plan view of the flatly developed diaper 1a, the leg elastic members 28 extending along the side edges of the bodily fluid-absorbent structure 22 in the front-back direction A respectively intersect with the front elastic region 71 and the rear elastic region 72 provided in the innermost sheet member 21 to be contractible in the transverse direction B. In addition, as will be apparent from Fig. 1, both the front elastic region 71 and the rear elastic region 72 are substantially continuous in the circumferential direction between the respective side edges 7a, 7a and 8a, 8a of the front and rear waist regions 7, 8 joined together at the joints 9. Consequently, the front and rear elastic regions 71, 72 in the vicinity of the respective side edges 7a, 7a and 8a, 8a cooperate with the elastic members 28, 28 intersecting with the front and rear elastic regions 71, 72 to define elastic regions adapted to surround the wearer's legs.

It should be noticed here that the innermost sheet member 21 is non-elastic in a bottom 75 of the crotch region 6 and therefore the width of this bottom 75, i.e., the dimension of the bottom 75 in the transverse direction B is never reduced due to the innermost sheet member 21. With the diaper 1 having such crotch region 6 put on the wearer's body, the side edges 27 of the crotch region 6 kept in tight contact with the wearer's inguinal region is not subject to a force in the transverse direction B functioning to move these side edges 27 of the crotch region 6 inward from the inguinal region. Consequently, the preventive effect of the diaper 1 against sideways leakage of bodily fluids is further improved.

Fig. 4 is a view similar to Fig. 3, illustrating one preferred embodiment of the present invention. In the flatly developed diaper 1a shown by Fig. 4, the rear end 52c of the second sheet 52 adapted to be contractible in the transverse direction B lies between front opening 31 and one of the paired regions 57 and the front end 53b of the third sheet 53 lies between the rear opening 32 and the other of the paired regions 57. With the diaper 1 obtained from such flatly developed diaper 1a put on the wearer's body, the respective portions of the front elastic region 71 and the rear elastic region 72 defining the front and rear waist regions 7, 8 are stretched in the waist-surrounding direction and the respective portions partially defining the crotch region 6 are stretched circumferentially of the respective legs. The respective peripheral edges 76, 77 of the front opening 31 and the rear opening 32 lie over all in the front elastic region 71 and the rear elastic region 72 and, with the diaper 1 put on the wearer's body, these peripheral edges 76, 77 closely fit over all to the wearer's skin. In the flatly developed diaper 1a, the non-elastic region 73 is defined between the paired regions 57 of the innermost sheet member 21. With the diaper 1 put on the wearer's body, the bottom 75 of the crotch region 6 having the side edges 27, 27 tightly retained by the wearer's inguinal region would not contract in the transverse direction B and the side edges 27, 27 would not be disengaged from the inguinal region.

As will be apparent from Figs. 3 and 4, in the second and third sheets 52, 53, the rear end 52c extends in the transverse direction B between the front opening 31 and one of the paired joints 37 (See Fig. 2), i.e., one of the paired regions 57 or across the front opening 31 and the front end 53b extends in the transverse direction B between the rear opening 32 and the other of the paired joints 37 or across the rear opening 32. In other words, the rear end 52c extends in the transverse direction B between a top 31t of the front opening 31 as viewed in the vertical direction C and one of the paired joints 37 and the front end 53b extends in the transverse direction B between a top 32t of the rear opening 32 and the other of the paired joints 37.

In the flatly developed diaper 1a of Fig. 4, in order to enhance contractile force of the second sheet 52 and the third sheet 53 along the peripheral edge of the waist-opening 11 when the diaper 1 is put on the wearer's body, a plurality of thread-like elastic members 78, 79 are attached under tension to the first sheet 51. In the flatly developed diaper 1a according to this embodiment, adhesive 41 used to bond the innermost sheet member 21 to the inner sheet 23 extends across the crotch region 6 to the both side edges 27, 27. In this case, the front body waste retaining sub-space 25a and the rear body waste retaining sub-space 25b are sealed to each other over all in the transverse direction B.

While the innermost sheet member 21 has the same planar shape as that of the first sheet 51 according to the illustrated embodiments, such innermost sheet member 21 may be replaced by an alternately formed innermost sheet member 21 comprising a first sheet 51 defining the crotch region 6 or a part of the crotch region 6 and a second sheet 52 defining the front waist region 7 or the front waist region 7 and a part of the crotch region 6.

While, in the diaper 1, the innermost sheet member 21 is preferably liquid-impervious and more preferably air-permeable and liquid-impervious, sheets having properties different one from another may be combined one with another to obtain the innermost sheet member 21 which is liquid-pervious in the crotch region 6 or in a part of the crotch region 6 and liquid-impervious in the front and rear waist regions 7, 8. For example, the first sheet 51 constituting the innermost sheet member 21 may be formed of a nonwoven fabric of thermoplastic synthetic fibers or a thermoplastic synthetic resin film. Alternatively, a laminated sheet of nonwoven fabric layers or a laminated sheet of nonwoven fabrics and films may be used as the first sheet 51. The second and third sheets 52, 53 may be formed, for example, of an elastically stretchable and contractible nonwoven fabric containing elastic threads such as urethane threads or an elastically stretchable and contractible film of natural or synthetic rubber. The inner sheet 23 may be formed, for example, of a liquid-pervious nonwoven fabric, a thermoplastic synthetic resin film or a composite sheet consisting of a nonwoven fabric and a film laminated one on another. The outer sheet 24 may be formed, for example, of a liquid-impervious nonwoven fabric, a thermoplastic synthetic resin film or a composite sheet consisting of a nonwoven fabric and a film laminated one on another.

## Claims

1. A disposable pants-type diaper having a front-back direction, a transverse direction and a vertical direction extending orthogonally one to another, a crotch region, a front waist region extending forward from said crotch region and a rear waist region extending rearward from said crotch region wherein said crotch region includes a bodily fluid-absorbent structure comprising a bodily fluid-absorbent core sandwiched between a liquid-pervious inner sheet and a liquid-impervious outer sheet and said disposable pants-type diaper is formed in said crotch region with a separator serving to separate urine and feces from each other, said disposable pants-type diaper being **characterized in that:**
said bodily fluid-absorbent structure has side flaps formed outboard of said core in said transverse direction and end flaps formed outboard of said core in said vertical direction;
the innermost sheet member joined to said side flaps and said end flaps to be interposed between said pants-type diaper wearer's skin and said inner sheet and to extend in said crotch region and said front and rear waist regions is partially spaced from said inner sheet in said vertical direction to define a body waste retaining space and said innermost sheet member is formed with a front opening allowing urine to flow into said body waste retaining space and a rear opening allowing feces to move into said body waste retaining space;
a region of said innermost sheet member extending between said front opening and said rear opening is folded along a fold line extending in said transverse direction and defined by a center line bisecting a dimension of said diaper in said transverse direction with a skin-facing surface of said innermost sheet member inside so that said region extending between said front and rear opening and folded to face itself and partially bonded together and said innermost sheet member extends downward from this bonded region toward said inner sheet in said vertical direction to form said separator; and
said innermost sheet member further includes elastic regions adapted to be elastically contractible and respectively defined between said region facing itself in said front-back direction and partially bonded together and a top of said front opening as viewed in said vertical direction and between said region facing itself in said front-back direction and partially bonded together and a top of said rear opening as viewed in said vertical direction, on one hand, and a non-elastic region defined by a portion extending downward from said bonded regions, on other hand.

2. The diaper defined by Claim 1 wherein said elastic regions extend in said transverse direction in said crotch region and joined under tension to said side flaps of said bodily fluid-absorbent structure.

3. The diaper defined by Claim 1 or 2 wherein said innermost sheet member is pant-shaped.

4. The diaper defined by any one of Claims 1 through 3 wherein said side flaps are provided with elastic members extending in said front-back direction attached under tension thereto and said elastic regions intersect and overlap said elastic members.

5. The diaper defined by Claim 4 wherein said elastic regions and said elastic members are bonded together in regions of intersecting and overlapping.
